# EUROPEAN PATENT SPECIFICATION

(11) **EP 2 459 319 B1**
(45) Date of publication and mention of the grant of the patent: **21.01.2015**
(21) Application number: 10774423.7
(22) Date of filing: 11.05.2010
(51) Int. Cl.: B05B 17/06, A61M 11/00, B06B 1/06, H03H 9/145

(54) **MICROFLUIDIC APPARATUS FOR THE ATOMISATION OF A LIQUID**
MIKROFLUIDISCHE VORRICHTUNG ZUR ATOMISIERUNG EINER FLÜSSIGKEIT
APPAREIL MICROFLUIDIQUE POUR L'ATOMISATION D'UN LIQUIDE

(30) Priority: 11.05.2009 AU 2009902063
(43) Date of publication of application: 06.06.2012
(73) Proprietor: Monash University, Clayton, VIC 3168 (AU)
(72) Inventor: FRIEND, James, Oakleigh East Victoria 3166 (AU); YEO, Leslie, Malvern Victoria 3144 (AU); MORTON, David, Williamstown Victoria 3016 (AU); McINTOSH, Michelle, Warrandyte Victoria 3113 (AU); QI, Aisha, Clayton South Victoria 3169 (AU); HO, Jenny, Clayton, Victoria 3800 (AU); RAJAPAKSA, Anushi, Victoria 3168 (AU)
(74) Representative: Rooney, John-Paul
(86) International application number: PCT/AU2010/000548
(87) International publication number: WO 2010/129994

(56) References cited:
- EP-A1- 0 844 027
- EP-A1- 1 952 896
- WO-A1-2007/135409
- DE-A1- 19 533 297
- JP-A- 2008 073 570
- JP-A- 2008 104 966
- US-A- 4 049 982
- US-A1- 2002 079 987
- US-B1- 6 325 475
- US-B1- 6 407 650
- PATENT ABSTRACTS OF JAPAN & JP 2008 104974 A (MATSUSHITA ELECTRIC WORKS LTD) 08 May 2008

## Description

### TECHNICAL FIELD

The present invention is directed to a microfluidic apparatus for the atomisation of a liquid. While the invention is described with respect to its use as a pulmonary delivery apparatus, it is to be appreciated that the invention is not restricted to this application, and that other applications are also envisaged.

### BACKGROUND TO THE INVENTION

Gene therapy represents a new paradigm of therapy for diseases, where the disease is treated at the molecular level by restoring defective biological functions or reconstituting homeostatic mechanisms within cells. Effective gene therapy requires that the Deoxyribonucleic acid (DNA) successfully accesses the target cell, is taken up for internalisation into the cell, is trafficked through the cell after escaping the degradative pathway to the nucleus, to subsequently be transcribed and translated to produce a desired gene product.

The lung is an important target for gene delivery because aerosol delivery is a non-invasive technique and can directly target the vast surface area of the lung. Plasmid DNA (pDNA) can be introduced into the lung by aerosol inhalation. However, delivery efficiency and durability of the gene vectors to comply with stringent requirements are critical areas for this approach to be successful. Potential obstacles for current pulmonary delivery devices include the retention of the supercoiled structure of the plasmid in the aerosols to retain its transfecting ability and to comply with regulatory requirements on product quality, and to produce aerosol particles with appropriate sizes for optimal delivery to lung surfaces.

Numerous studies have been undertaken in order to determine the feasibility of pulmonary delivery devices in delivering non-complexed pDNA to the lungs. Unfortunately, the supercolled tertiary structures of pDNA with sizes larger than 5 kilo-base pairs (kbp) have been found to be severely sheared into open circular and fragmented DNA by hydrodynamic shear and shock waves during nebulization in jet and ultrasonic nebulizers. The emergence of new devices such as mesh nebulizers, electrohydrodynamic (EHD) devices and miniaturized nebulization catheter devices have been said to offer greater aerosolization efficiency, and preserve the integrity of pDNA in the aerosols. However, these devices require more clinical studies to demonstrate this.

Inhalation therapy has become the treatment of choice for asthma and chronic obstructive pulmonary disease (COPD). Unlike oral dosing, inhalation therapy allows a high concentration of a drug to be administered and targeted directly to local inflammation sites within the lung, thereby enabling lower total dosages, reduction in systemic side effects, and potentially hastening the onset of action of the drug. Metered Dose Inhalers (MDIs) and Dry Powder Inhalers (DPIs) are commonly used for bronchodilator administration for asthma and COPD therapy; the patient inhales a pre-metered dose in a single forced inspiratory manoeuvre. There is a lively debate among researchers, however, in deciding whether MDIs or DPIs are the most effective or if continuous nebulization to a patient undergoing repeated tidal breathing for a period up to several minutes is required. Though the debate continues, critical factors in making such decisions are generally based on clinical judgements, taking into consideration such factors as dose level, drug efficacy and safety profile, patient age group, disease severity, ease of administration, and cost.

Nebulizers are capable of delivering more drug than current MDIs and DPIs because they operate over a longer period. Moreover, nebulizers do not require coordination skills from the patient, unlike MDIs, and do not require patient actuation via inhalation, unlike DPIs. Nebulizers are commonly used in acute cases of COPD or severe asthma attacks where the patient is unable to self-medicate. For this same reason, nebulizers may be more appropriate for paediatric and geriatric patient populations.

Historically, nebulizers have been large, cumbersome, less portable and more expensive than MDIs or DPIs. Furthermore, conventional nebulizers generally have low dose efficiencies; although more drug may be delivered into an aerosol, much of the aerosolized drug is subsequently wasted because:
1. aerosols are generated continuously, wasting drug as the patient exhales against the nebulizer's output,
2. the aerosols have polydisperse size distributions, with a significant fraction of droplets too large for deep lung deposition, and since
3. nebulizers typically have a large internal residual volume.

For inhalation therapy to be most effective, the droplet's aerodynamic behaviour (governed by Stokes' law) Is of fundamental importance. For deep lung deposition, an aerodynamic diameter less than 5 µm or preferably 3 µm Is considered appropriate, such that the aerosol can avoid inertial impaction in the oropharyngeal region. For deposition higher up in the airways, a larger aerodynamic diameter may be preferred. As a result, the aerosol droplet size is crucial to the efficacy of Inhalation therapy, and therefore an ideal device capable of efficiently delivering high doses of a drug would permit precise control of the droplet size distribution and preferably offer large atomisation rates to deliver the desired dosage in as short a time period as possible to minimize patient distress and inconvenience.

Nebulization technology has rapidly progressed in recent years, with new methods that utilize ultrasound and electrohydrodynamic atomisation, allowing greater control over the atomisation process to provide aerosols with reduced spreads of polydispersity and with droplet size tuning capability. Furthermore, these methods may be miniaturized, offering an attractive alternative to the large and cumbersome nebulizers that are currently available commercially. Unfortunately, these methods have inherent limitations. For example, electrohydrodynamic atomisation is restricted to high voltage operation - typically several kilovolts - raising safety and reliability issues in consumer use. Various types of ultrasonic atomisation have been devised over the years, and the most common systems use a bath of liquid from which a piezoelectric disc generates an aerosol plume. These ultrasonic nebulizers are also relatively large in size, have limitations on output and size control, and often precipitate the solubilized drug onto the atomisation reservoir walls due to solvent evaporation, wasting the drug and requiring regular cleaning by the user. More recent designs using meshes for nebulization offer better portability, dosage rates, and aerosol monodispersity. The mesh has chemically or laser-cut microscopic holes, forming thousands of orifices that generate droplets under irradiation by ultrasound, although these meshes are prone to clogging, which significantly reduces throughput. In the context of these past and current technologies, a small, portable, reliable, and relatively cost-effective device remains out of reach, especially one that can effectively generate non-agglomerating droplet size distributions which are suitably monodisperse and less than 5-10 µm in diameter.

EP 1 952 896 A1 discloses a droplet dispenser having features as in the preamble of the appended claim 1.

US 6407650 B1 and US 2002/079987 A1 are also relevant.

### SUMMARY OF THE INVENTION

With this in mind, the present invention provides an apparatus for the atomisation of a liquid including:
a piezoelectric substrate having at least one working surface; at least one electrode supported on the piezoelectric substrate; the electrode being an elliptical, electrode width controlled single phase unidirectional transducer (EWC-SPUDT);
a signal generating means for applying an ultrasonic signal to said electrode for generating a surface acoustic wave (SAW) in the working surface of the piezoelectric substrate; and
a liquid delivery arrangement including a wick in contact with the working surface for delivering liquid thereof,
wherein liquid delivered to the working surface by the wick is atomised by SAW irradiation.

The electrode is configured as EWC-SPUDT as this configuration gives the largest surface acoustic wave intensity into the liquid sitting on the substrate compared to straight standard and SPUDT-style interdigital transducer electrodes, circular EWC-SPUDTs, and other configurations known currently. For a given input power, the elliptical EWC-SPUDT therefore offers the best atomisation performance of these various configurations, and the width and ellipticity of the EWC-SPUDT so chosen is preferably tailored to the size of the liquid drop sitting upon the substrate. The relationship between the size of the droplet and the exit aperture (width) of the EWC-SPUDT depends on the liquid properties, but the ratio of drop diameter to exit aperture is preferably between 0.5 and 2.

Preferably more than one EWC-SPUDT may be used. For example, two may be used on very anisotropic piezoelectric materials like lithium niobate (class [3m]), while more can be used on less anisotropic materials like ZnO, AIN, or PZT.

The frequency of atomisation is preferably between 10MHz and 250MHz, depending on the liquid to be atomized, and this defines the electrode finger width and the gaps between them in the EWC-SPUDT.

The wick of the liquid delivery arrangement may be provided by at least one paper strip or string, with the liquid being delivered through capillary action. Other types of porous material providing a similar capillary action are also envisaged, for example cloth fabric, or other hydrophilic materials.

The liquid delivery arrangement may preferably also include a liquid reservoir container for containing the liquid to be delivered to the apparatus. The wick may extend from the surface of the piezoelectric substrate all the way to the interior of the liquid reservoir. Alternatively, a capillary tube may extend from the liquid reservoir, the wick receiving the liquid via this capillary tube. The liquid reservoir itself may be provided by a replaceable vial.

The capillary tube may preferably be of various shapes (bent to accommodate device design, for example), and placed with wick in a variety of orientations to contact the substrate and form the droplet. The capillary tube may however be omitted, with only the wick between the liquid reservoir and substrate.

A driver circuit preferably controls the apparatus based on the measurement of the user's breathing and safety interlocks on the apparatus as commanded through the user interface.

The use of surface acoustic wave (SAW) atomisation has a number of advantages over ultrasonic nebulization. Surface acoustic waves are MHz to GHz-order, transverse-axial polarized elliptical electroacoustic waves with displacement amplitudes of just a few nanometers. Here, they are generated on and traverse the surface of the piezoelectric substrate. Unlike typical ultrasound, which is a bulk phenomenon, the SAW is confined close to the substrate surface, its amplitude decaying rapidly over a depth of four to five wavelengths (several hundred microns) into the substrate material. Compared to conventional ultrasonic atomizers that consume power on the order of 10 W, the apparatus according to the present invention may only consume between 0.5-3 W since most of the energy is contained within a localized region close to the surface of the substrate and hence can be transmitted Into the liquid much more efficiently than ultrasound. Moreover, the apparatus and power supply may be small showing the potential of the apparatus for portable applications. Moreover the 10-500 MHz order frequency employed in the apparatus is significantly higher than the 20 kHz-3 MHz frequency range of typical ultrasonic devices, induce vibrations with a period much shorter than the molecular relaxation time scale associated with large molecules in liquids, and thus the risk of denaturing molecules or lysing cells is greatly reduced. Further, as the frequency is increased, the power required to induce cavitation increases far beyond what is needed for atomisation, eliminating the effect of cavitation-induced lysis or shear in the apparatus.

In preliminary experiments conducted by the inventors on the atomisation characteristics of a salbutamol ethanol/octanal solution, a mean aerosol diameter of 2.84 ± 0.14 µm was achieved using SAW atomisation. Salbutamol is a drug used in the treatment for asthma, and the aerosol diameters achieved are well within the optimal range for deep lung deposition.

Though the amplitude of a SAW is only a few nanometers, the acceleration of the surface is incredibly high (10⁷ m²/s) because of the high operating frequency. Therefore, when a SAW is transmitted into a liquid drop placed upon the substrate, it is able to not only form capillary waves across the free surface of the droplet but also drives it to break up into a mist of droplets with an average, controlled diameter of 1-10 µm. Micro and nanoparticles may be formed via controlled evaporation of these droplets, but irrespective of the desired product the challenge is in maintaining a relatively stationary free liquid surface on the SAW apparatus as an atomisation source.

The liquid delivery arrangement according to the present invention resolve these issues. Using a wick to siphon liquid from a liquid reservoir to the working surface of the apparatus provides it with continuous flow without pumping. The wick therefore permits atomisation without affecting the performance of the apparatus and offers a constraint sufficient to retain a stable meniscus outside the peripheral edge of the wick on the surface as noted in recent experiments by the inventors using such a paper wick. In these experiments, it was found that the meniscus is constantly replenished by liquid passing from the paper wick by liquid passing from the paper and provides a surface for the formation and destabilization of a capillary wave to eject the aerosol. The aerosol is ejected at an angle dependent upon the shape of the meniscus, itself dependent upon the power used to generate the SAW. The fluid absorption rate of the paper defines the upper limit in flow rate for the apparatus.

### BRIEF DESCRIPTION OF THE DRAWINGS

It will be convenient to further describe the invention with respect to the accompanying drawings which illustrates a preferred embodiment of the apparatus according to the present invention. Other embodiments of the invention are possible, and consequently, the particularity of the accompanying drawings is not to be understood as superseding the generality of the preceding description of the invention.

In the drawings:
Figure 1 is a schematic view of an apparatus for the atomisation of a liquid according to the present invention;
Figure 2 is a schematic plan view of an interdigital electrode for the apparatus of Figure 1;
Figure 3 is a plot showing the cumulative size distribution obtained from the atomisation of 100 ug ml⁻¹ pDNA added with different weight ratio of glycerol using 30 MHz SAW with SPUDTs at 3 W, measured by laser diffraction. □, ◊, ○ and * represent 0, 10, 20 and 40 % w/w of glycerol respectively;
Figure 4 is a plot showing the volume-based droplet size distribution of pDNA at 10 (○), 50 (□) and 100 (×) ug ml⁻¹ indicate narrow monodisperse distributions with *D*ᵥ₅₀ = 3.85 ± 0.02, 4.82 ± 0.13 and 3.81 ± 0.06 µm, respectively;
Figure 5 is a table showing aerosol diameters from the atomisation of different pDNA formulations using 30 MHz SAW with SPUDTs at 3 W;
Figure 6 is a structural analysis of pVR1020-MSP4/5 before and after SAW atomisation by AFM imaging in air: (a) supercoiled structure of 5.6 kbp pre-atomized (control) pDNA and (b) post-atomisation by 30 MHz SAW;
Figure 7 shows the result of ethidium bromide agarose gel electrophoresis for the assessment of the naked pDNA structural integrity before and recovered after SAW atomisation. Lane M: 1 kbp DNA ladder; lanes 1 and 4: control pDNA prepared at concentration of 85 ug ml⁻¹ and 50 ug ml⁻¹, respectively; lanes 2 and 5: recovered 85 ug ml⁻¹ and 50 ug ml⁻¹ pDNA atomized with 30 MHz SAW,
   respectively; lanes 3 and 6: recovered 85 ug ml⁻¹ and 50 ug ml⁻¹ pDNA atomized with 20 MHz SAW, respectively. Each lane was loaded with 200 ng pDNA and representative gels from three independent experiments are shown. Arrows indicate the position of open circular (OC) and supercoiled (SC) forms of pDNA;

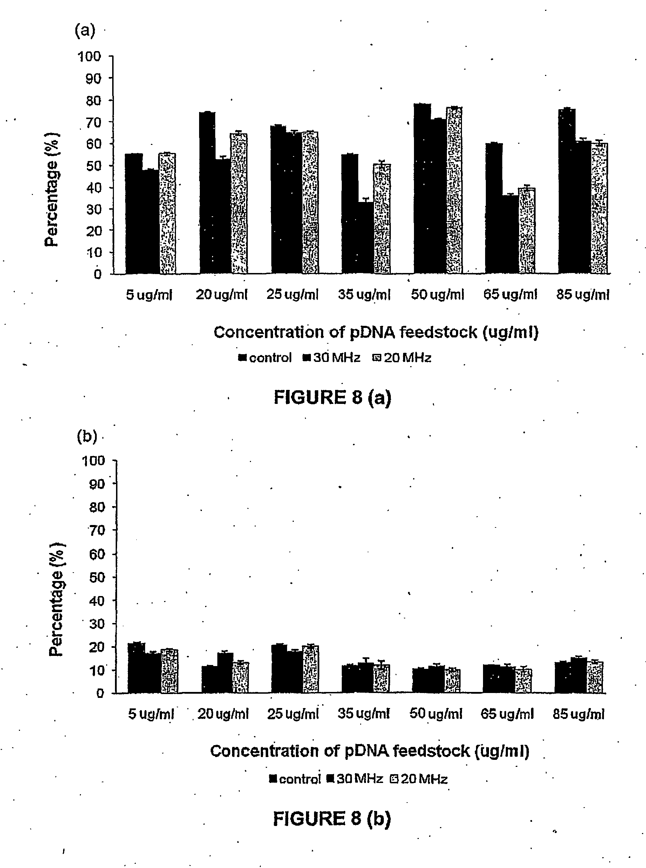

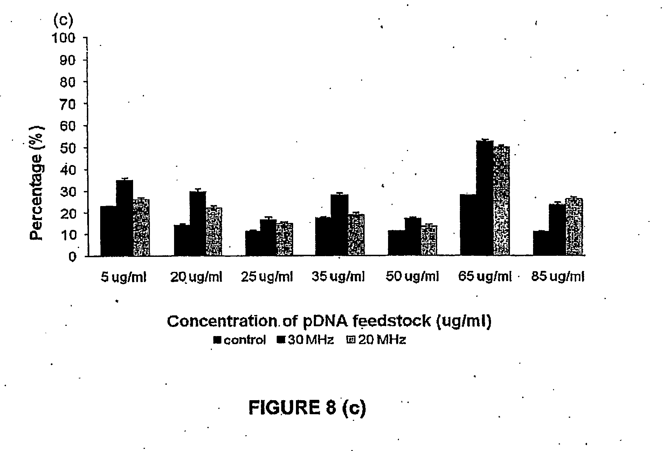

Figure 8 is a statistical analysis for percentage of (a) supercoiled, (b) open circular and (c) fragmented pDNA before and after SAW atomisation. The amount of supercoiled, open circular and fragmented DNA was assessed with results from agarose gel electrophoresis. The results are the mean of triplicate atomisation runs ± standard deviation; and

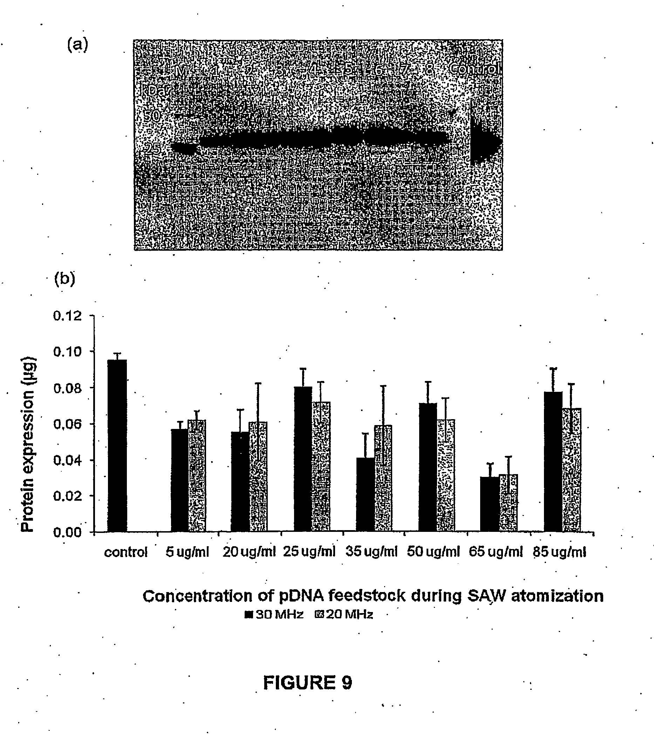

Figure 9 shows (a) Western blot detections of PyMSP4/5 expressed in COS-7 cells at 48 h post-transfection. Lane M: marker shown molecular mass standards (kDa) on the left; lanes 1 - 3: the cells transfected with DNA recovered from 35, 50 and 85 ug ml⁻¹ DNA atomized with 30 MHz SAW; lanes 4 - 8 the cells transfected with DNA recovered from 25, 35, 50, 65 and 85 ug ml⁻¹ DNA atomized with 20 MHz SAW. (b) Statistical analysis of *in vitro* transfection efficiency of naked pDNA recovered from SAW atomisation. The results are the mean of triplicate atomisation runs ± standard deviation.

### DETAILED DESCRIPTION OF THE INVENTION

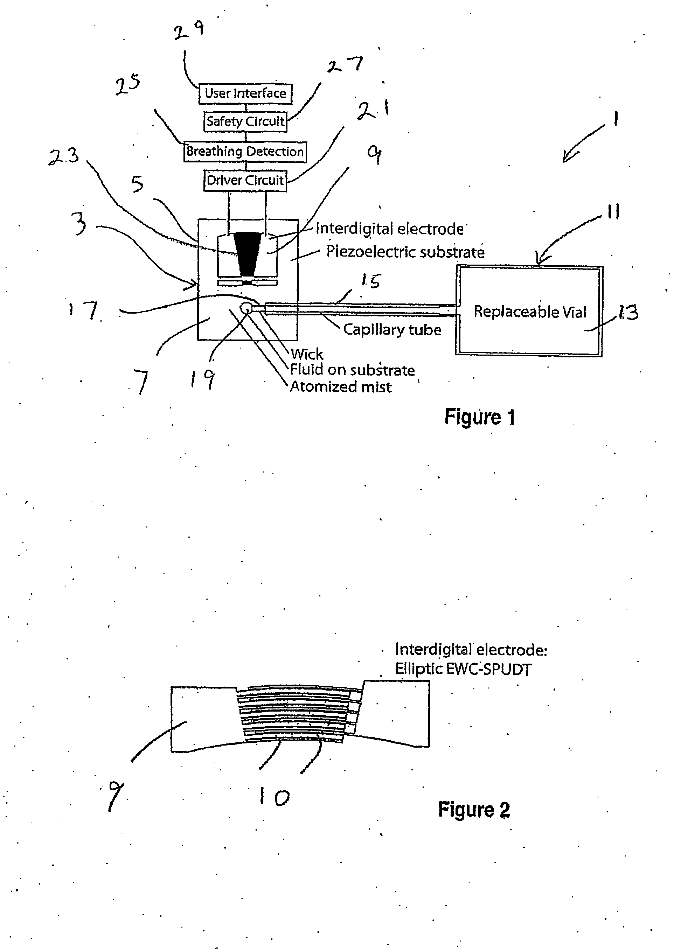

As shown in Figure 1, the apparatus 1 according to the present invention includes a transducer element 3 having a piezoelectric substrate 5 providing a working surface 7 for the apparatus.

Supported on the working surface 7 is an interdigital electrode 9 it has been found that the preferred electrode configuration is an elliptical electrode with controlled signal phase unidirectional transducer (EWC-SPUDT). It has been found that such an electrode provides the largest SAW intensity to the liquid delivered to the working surface 7. The interdigital electrode 9 is better shown in Figure 2 which shows the electrode having a series of interlaced elliptically curved electrode fingers 10. The width and gap of these fingers may preferably be set to be a quarter of the SAW wavelength.

A liquid delivery arrangement 11 is provided for delivering liquid to the working surface 7. This arrangement includes a liquid reservoir 13 which may be in the form of a replaceable vial. A capillary tube 15 extends from the liquid reservoir 11 for supplying liquid to a wick 17 having one end thereof in contact with the working surface, the opposing end thereof being located within the capillary tube 15. The liquid delivery arrangement therefore enables a liquid meniscus 19 to be formed on the working surface 7 for atomisation.

The wick 17 can be in the form of a strip or string of paper, with one end-of the wick being in contact with the working surface 7. As liquid is supplied to the working surface 7 through the wick 17, the meniscus 19 is formed between the end of the wick 17 and the working surface 7. This meniscus 19 is continuously replenished by liquid passing from the wick 17 and provides a surface for the formation and destabilisation of a capillary wave to eject the atomised droplets therefrom.

A driver circuit 21 applies an ultrasonic signal to the interdigital electrode 9, typically between 10 to 250MHz, thereby resulting in a SAW 23 being generated in the working surface 7. The interaction of the SAW 23 with the liquid meniscus 19 results in an atomised mist of droplets.

The driver circuit 21 is controlled based on measurements of the user's breathing by a detection sensor 25, and by safety interlocks in a safety circuit 27 control through a user interface 29.

The apparatus 1 of the present invention has various applications and could be used for inhalation gene therapy and vaccination using genetic biomolecular materials, which could include plasmid DNA, siRNA, protein molecules, etc. Other applications may include DNA encapsulation, DNA stretching/hybridization and DNA micro-array printing.

The inventors have conducted experiments to assess the feasibility of SAW atomisation as an aerosol delivery platform for pulmonary genetic therapeutics, especially with shear-sensitive naked (non-complexed) pDNA encoded with desired genes. These experiments examined the structural integrity of the pDNA following delivery, the size distribution of the aerosol droplets, and the physical stability and *in vitro* transfection efficiency of pDNA recovered after SAW atomisation. The plasmid VR1020-PyMSP4/5 was used in the experiments. The following experimental results were obtained.

### i) Aerosol size distribution

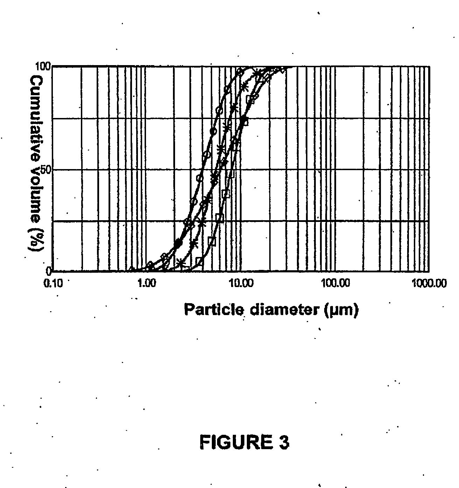

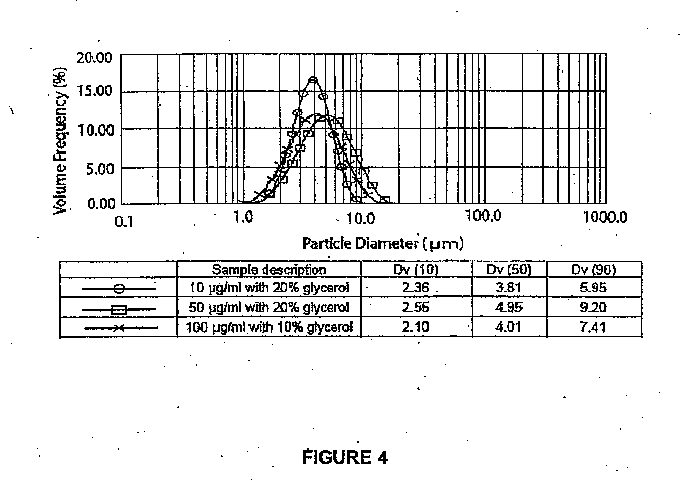

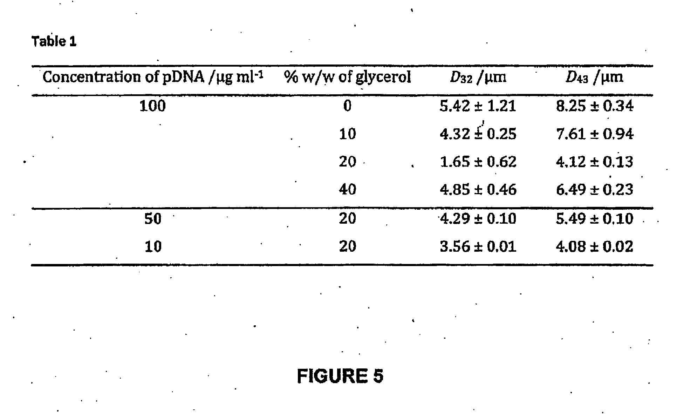

It has been previously noted that droplets with aerodynamic diameter below 5 µm are desired for efficient deep lung deposition. Generation of aqueous pDNA at droplet size smaller than 5 µm has proved challenging due to the high surface tension of water. A formulation of pDNA containing glycerol was chosen in these experiments to minimize the surface tension and to increase the viscosity of the atomized liquid film. Glycerol has been included in pharmaceutical formulations for the delivery of some drugs, but with concentrations less than 40 % w/w. After careful consideration of toxicity and the size characteristic of generated aerosols, a narrow monodisperse aerosol mist with droplet size < 5 µm was reliably obtained. Fig. 3 shows the aerosol cumulative size distributions for pDNA at concentration of 100 ug ml⁻¹ and added with different weight ratio of glycerol to manipulate the viscosity of liquid film (□,◊, ○ and * represent 0, 10, 20 and 40% of glycerol respectively in Fig. 3). It was found the D₄₃ (DeBrouckere mean diameter) dropped from 8.25 ± 0.34 µm to 4.12 ± 0.13 µm when 100 ug ml⁻¹ pDNA was added with 20 % w/w of glycerol. However, the *D*₄₃ further increased to 6.49 ± 0.23 µm when added with 40 % w/w of glycerol. The inconsistence is believed to be caused by the evaporation of aerosols. The size measurement was taking place about 7 cm above the SAW device, therefore, a certain level of evaporation occurred. When .40 % w/w glycerol was added, the solution is too viscous to evaporate; the aerosol size thus appeared to be larger compared to solutions with less glycerol. Furthermore, considering the fact that less glycerol introduces less toxicity, it is reasonable to take the solution with 20% glycerol as the optimal option for further investigations. Glycerol's surface tension and viscosity are 64 mNm⁻¹ and 1.5 Pa.s, respectively, which are much influential upon the droplet diameter as compared to DNA (viscosity < 3 mPa.s). The aerosol size distribution for pDNA at several concentrations added with 20 % w/w glycerol (see Fig. 4) further agreed with this prediction and also the scaling relationship we have developed in the above noted equation for SAW atomisation, The results for aerosol diameter are summarized In Table 1 (see Fig. 5). Therefore, we can finely tune the droplet size < 5 µm for pDNA using SAW atomisation and confident that the pDNAs are transport to alveolar region. These results are encouraging as compared with a previous nebulization catheter device which only managed to generate naked pDNA aerosol with size around 33 ± 2 µm.

### ii) Physical stability of pDNA molecules driven by SAW

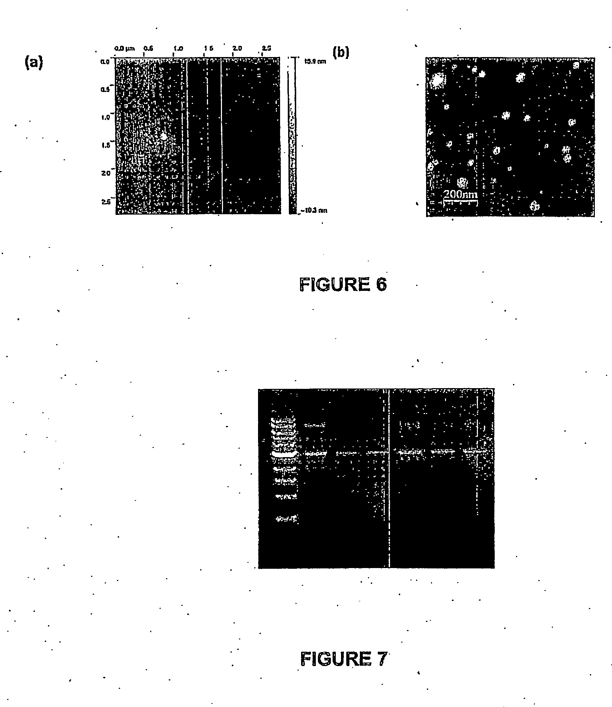

The supercoiled structure of pDNA is maintained by the covalent bonds in the phosphodiester backbone. The torsional energy stored in the native compact supercoiled plasmid will be released upon the bond scission on either end of the double strands, and take a relaxed open circular form. Further cleavage of strands at the same location on the opposite strand of the DNA helix will form a linear polynucleotide. This full-length linear molecule will be fragmented into a smaller size if the entire double strand breaks. The AFM imaging of the pVR1020-MSP4/5 indicates the tightly twisted supercoiled geometry before SAW atomisation (see Fig. 6a). Integrity of pDNA molecules in the generated aerosols is crucial in evaluating the feasibility of a device for the aerosol delivery of pDNA. The retainment of supercoiled and covalently close circular (open circular) form of plasmid DNA is important to achieve maximum biological activity and to comply the regulatory requirements for plasmid products. Different concentrations of pVR1020-MSP4/5 were atomized through the 20 MHz and 30MHz SAW, and collected for analysing of integrity using agarose gel electrophoresis (see Fig. 7) and AFM (see Fig. 6b). Interestingly, agarose gel electrophoresis revealed that the SAW atomisation process highly preserved the unprotected naked pDNA. The change in supercoiled, open circular and fragmented DNA structures prior to and after atomisation is presented in Fig. 8. In most of the cases, more than 90% of the initial supercoiled DNAs were maintained (Fig. 8a) and the degradation is majority governed by the integrity of pDNA feedstock during SAW atomisation, *i.e.* the percentage of supercoiled, open circular and particularly the fragmented. DNA that were subjected to atomisation. This phenomenon was observed for samples at concentration 5, 35 and 65 µg ml⁻¹, which have around 60 % and 20 % of supercoiled and fragmented DNA respectively in the feedstock, and this damage was due to the early stages of plasmid purification. The key parameter affecting the shear sensitivity of DNA molecules Is Its hydrodynamic diameter. The linear double stranded DNA molecules are very susceptible to flow induced stresses as studied in the past and minor differences in its characteristic dimension will have large effects on its response to fluid flow stresses.

During SAW atomisation, Isoforms of a plasmid present in the initial preparation (purification process) such as open circular and linear are more sensitive to hydrodynamic forces due to their length. Increasing in length and hydrodynamic diameter of plasmids (open circular and linear) due to the changes in tertiary structure lower the shearing threshold, and the relaxed forms will subject to larger forces which cause fragmentation. Experimental and theoretical studies summarizes that stretching hydrodynamic forces > 300 pN will cause irreversible strand separation and nick formation even before reaching sufficient forces to break covalent bonds (1600 - 5000 pN). The displacement velocity along substrate surfaces during SAW propagation is typically 1 m s⁻¹ regardless the excitation frequency. The surface acceleration is extremely high (10⁷ - 10⁸ m² s⁻¹) although the amplitude of the acoustic wave only around 10 nm. Such huge acceleration, when being transmitted into the liquid placed upon substrate surface, give rise to a bulk circulation within the liquid, which in turn, induces strong destabilized capillary waves on the free liquid surface..When the supplying power is sufficient, capillary wave eventually breaks up and forms aerosol droplets at the liquid-air interface. Balance between the viscous force arising from the *acoustic streaming* and stabilizing capillary forces gives the instability threshold at the onset of SAW atomisation. If the relaxed forms of plasmid located close to the free liquid surface, the hydrodynamic forces are more intense at the crest of the conically shaped end and give rise to higher damage.

On other aspect, the degradation of pDNA was found slightly dependent on the concentration of DNA during atomisation. When both of the 50 and 85 ug ml⁻¹ sample have similar amount of integrity (> 75 % of supercoiled DNA) during atomisation, the 85 ug ml⁻¹ sample was found to has larger extent of degradation (20 % degradation) as compared with 50 ug ml⁻¹ sample (< 9 %). The same outcome was found for less integrity samples; higher concentration samples have degradation greater than 35 % (35 and 65 ug ml⁻¹ samples) while only around 13 % of degradation for 5 ug ml⁻¹ sample.

Conventional acoustically driven method at lower kilohertz (kHz) order frequencies cause substantial loss in initial supercoiled DNA structure specifically due to the cavitation streaming during formation, growth and subsequent collapse of bubbles. Indeed, DNA molecules within the solution act as nucleation sites for cavitation. SAW microfluidic actuation retains the benefits of using acoustic fields for driving fluid motion, namely, the large actuation speeds and the associated flow nonlinearities due to inertial forcing, while addressing the limitations that plague conventional ultrasonic methods. The high megahertz (> 10 MHz) order SAW vibrations facilitate fluid and particle manipulation at a much finer scale, and provide more energy efficient mechanism by concentrating the energy into the narrow surface region of fluid. These exceptional advantages avoid the damage of DNA since the shear gradient generated within such a short period of time is not sufficient to degrade the DNA. However, the higher DNA concentration solution can be pulled apart at multiple nucleation sites; eventually the solution become unstable and collapse, creating shocking waves and areas of extreme pressure and temperature. In general, the extent of degradation during SAW atomisation is far milder compared to conventional ultrasonic nebulisation (> 35 %) and vibrating mesh nebulizer (> 40 %).

### iii) Bioactivity of plasmids during in vitro transfection

Shearing forces generated in conventional nebulisation methods endanger the integrity of naked pDNA, and this led to marked reduction in transfection efficiency both *In vitro* and *ln vivo.* In the experiments, the *in vitro* transfection efficiency of SAW atomized pVR1020-MSP4/5 was investigated in immortalized African green monkey kidney cells (COS-7). The gene expression results are in agreement with the agarose gel electrophoresis observation - higher open circular and fragmented ratio post atomisation entailed with lower gene expression. As high as 84 % and 75 % of transfection efficiency were observed on the *in vitro* MSP4/5 gene expression of the recovered pDNA after 30 MHz and 20 MHz SAW atomisation, respectively, compared to the corresponding unatomized (control) pDNA at 48 h (see Fig. 9). The retention of the *in vitro* transfection efficiency offered additional evidence that the pDNA molecules are well protected during the SAW atomisation. Thus, SAW appears to be a useful and efficient alternative for aerosol delivery of shear sensitive biomolecules.

### iv) Conclusions of experiments

It has therefore been found that the present invention advantageously reduces the risk of denaturing molecules since the period to induce vibrations in the apparatus 1 by employing 10-100 MHz order frequency is much shorter than the molecular relaxation time scale of macromolecules in liquids. In addition, cavitation is largely absent when the frequency is increased beyond a few MHz thus eliminating the effect of cavitation-induced lysis or shear for shear-sensitive molecules such as naked pDNA encoded with desired genes. Further, the size of droplets generated by the apparatus 1 can be changed by about an order of magnitude In a few microseconds in a controllable fashion by switching from a standing-wave to travelling-wave. Hence, the present invention has significant advantages over ultrasonic medical nebulizers that represent the current state of the art.

Further, SAW microfluidic actuation retains the benefits of using acoustic fields for driving fluid motion, namely, the large actuation speeds and the associated flow nonlinearities due to inertial forcing, while addressing the limitations that plague conventional ultrasonic methods. The high megahertz (> 10 MHz) order SAW vibrations facilitate fluid and particle manipulation at a much finer scale, and provide more energy efficient mechanism by concentrating the energy into the narrow surface region of fluid. These exceptional advantages avoid the damage of DNA since the shear gradient generated within such a short period of time is not sufficient to degrade the DNA.

The SAW atomisation provided by the present invention is a viable means for generating aerosols of shear-sensitive biotherapeutics - plasmid DNA, and provides almost negligible denaturation of the supercoiled content. The present invention can be utilised to provide plasmid-laden aerosols which have a droplet size < 5 µm for optimal deep lung deposition and remain biological active with tests demonstrating successful gene expression in mammalian cells after SAW atomisation. The apparatus 1 of the present invention is therefore suitable as a pulmonary delivery platform for DNA molecules, proteins arid other blomolecules. Tests have also found that there is little damage to the mesenchymal stem cells, without hampering their viability, proliferation and differentiation after SAW irradiation. The low power requirement (as low as 1 W) compared with that required with conventional ultrasonic nebulizers allows the apparatus 1 to be miniaturised in a portable palm sized device, powered by battery and incorporated with advanced electronic detection and control for adaptive delivery.

The present invention provides an efficient and rapid process to generate biomolecule-laden aerosols with minimum disruption upon the innate structure of biomolecules, for example DNA biomolecules for gene therapy. This can advantageously minimise the waste of expensive molecules such as vaccines and drugs, and enable effective therapy as compared to all other atomisation processes which will destroy the biomolecules. The present invention provides a major breakthrough for aerosol gene delivery and provides great promise for non-viral gene therapy using a non-invasive approach.

Modifications and variations as would be deemed obvious to the person skilled in the art are included within the ambit of the present invention as claimed in the appended claims.

## Claims

1. An apparatus for the atomisation of a liquid including:
a piezoelectric (5) substrate having at least one working surface (7), at least one electrode (9) supported on the piezoelectric substrate (5),
a signal generating means (21) for applying an ultrasonic signal to said electrode (9) for generating a surface acoustic wave (SAW) (23) in the working surface (7) of the piezoelectric substrate (5); and
a liquid delivery arrangement (11) including a wick (17) in contact with the working surface (7) for delivering liquid thereof,
wherein liquid delivered to the working surface (7) is atomised by SAW irradiation,
**characterised by** the electrode (9) being an elliptical, electrode width controlled single phase unidirectional transducer (EWC-SPUDT).

2. An apparatus according to claim 1, wherein the wick (17) of the liquid delivery arrangement (11) is provided by at least one paper strip or string.

3. An apparatus according to claim 2, wherein the liquid delivery arrangement (11) further includes a liquid reservoir (13) for containing the liquid to be delivered to the apparatus.

4. An apparatus according to claim 3, further including a capillary tube (15) extending from the liquid reservoir (13), the wick (17) receiving the liquid via this capillary tube (15).

5. An apparatus according to any of claims 3-4 wherein the liquid reservoir (13) contains a nucleic acid composition.

6. An apparatus according to any of claims 3 to 4 wherein the liquid reservoir (13) contains salbutamol.

## Patentansprüche

1. Eine Vorrichtung zur Atomisierung einer Flüssigkeit, aufweisend:
ein piezoelektrisches Substrat (5) mit wenigstens einer Arbeitsfläche (7), wobei wenigstens eine Elektrode (9) von dem piezoelektrischen Substrat (5) getragen ist;
ein Signalerzeugungsmittel (21) zum Anlegen eines Ultraschallsignals an die Elektrode (9) zur Erzeugung einer Oberflächenakustikwelle (SAW) (23) in der Arbeitsfläche (7) des piezoelektrischen Substrats (5); und
eine Flüssigkeitszufuhranordnung (11) mit einem Docht (17) in Kontakt mit der Arbeitsfläche (7) zur Zufuhr von Flüssigkeit hierauf,
wobei die der Arbeitsfläche (7) zugeführte Flüssigkeit durch SAW-Anlage atomisiert wird,
**dadurch gekennzeichnet, dass** die Elektrode (9) ein elliptischer, elektrodenbreitengesteuerter einphasiger unidirektionaler Wandler (EWC-SPUDT) ist.

2. Eine Vorrichtung nach Anspruch 1, wobei der Docht (17) der Flüssigkeitszufuhranordnung (11) durch wenigstens einen Papierstreifen oder-faden gebildet ist.

3. Eine Vorrichtung nach Anspruch 2, wobei die Flüssigkeitszufuhranordnung (11) weiterhin ein Flüssigkeitsreservoir (13) zur Aufbewahrung der der Vorrichtung zuzuführenden Flüssigkeit enthält.

4. Eine Vorrichtung nach Anspruch 3, weiterhin mit einer Kapillarröhre (15), welche sich von dem Flüssigkeitsreservoir (13) aus erstreckt, wobei der Docht (17) die Flüssigkeit über diese Kapillarröhre (15) erhält.

5. Eine Vorrichtung nach einem der Ansprüche 3 bis 4, wobei das Flüssigkeitsreservoir (13) eine Nukleinsäurezusammensetzung enthält.

6. Eine Vorrichtung nach einem der Ansprüche 3 bis 4, wobei das Flüssigkeitsreservoir (13) Salbutamol enthält.

## Revendications

1. Appareil pour l'atomisation d'un liquide, comprenant :
un substrat piézoélectrique (5) ayant au moins une surface de travail (7), au moins une électrode (9) supportée sur le substrat piézoélectrique (5),
un moyen de génération de signal (21) pour appliquer un signal ultrasonore à ladite électrode (9) pour générer une onde acoustique de surface (OAS) (23) dans la surface de travail (7) du substrat piézoélectrique (5) ; et
un système de distribution de liquide (11) comprenant une mèche (17) en contact avec la surface de travail (7) pour délivrer le liquide de celui-ci,
dans lequel le liquide délivré sur la surface de travail (7) est atomisé par irradiation d'OAS,
**caractérisé en ce que** l'électrode (9) est un transducteur unidirectionnel monophasé à largeur d'électrode contrôlée elliptique (EWC-SPUDT).

2. Appareil selon la revendication 1, dans lequel la mèche (17) du système de distribution de liquide (11) est fournie par au moins une bande ou une ficelle de papier.

3. Appareil selon la revendication 2, dans lequel le système de distribution de liquide (11) comprend en outre un réservoir de liquide (13) pour contenir le liquide à délivrer à l'appareil.

4. Appareil selon la revendication 3, comprenant en outre un tube capillaire (15) s'étendant à partir du réservoir de liquide (13), la mèche (17) recevant le liquide par l'intermédiaire de ce tube capillaire (15).

5. Appareil selon l'une quelconque des revendications 3 à 4, dans lequel le réservoir de liquide (13) contient une composition d'acides nucléiques.

6. Appareil selon l'une quelconque des revendications 3 à 4, dans lequel le réservoir de liquide (13) contient du salbutamol.
